# EUROPEAN PATENT APPLICATION

(11) **EP 2 921 102 A1**
(43) Date of publication of application: **23.09.2015**
(21) Application number: 15159175.7
(22) Date of filing: 16.03.2015
(51) Int. Cl.: A61B 5/00, A61B 5/145, A61B 5/1455

(54) **BIOLOGICAL MEASUREMENT APPARATUS AND A BIOLOGICAL MEASUREMENT METHOD**

(30) Priority: 18.03.2014 JP 2014054505
(71) Applicant: Seiko Epson Corporation, Tokyo 163-0811 (JP)
(72) Inventor: Ikebe, Tomo, Nagano, 392-8502 (JP)
(74) Representative: Hoffmann Eitle

(57) **Abstract**

A biological measurement apparatus that can measure a biological component concentration with favorable precision is provided. This apparatus includes an infrared sensor that irradiates an arm with infrared light, receives infrared light that was reflected by the arm, and outputs light intensity data; a holding unit that holds the infrared sensor such that the infrared sensor comes into contact with the arm; a force sensor that detects the force with which the infrared sensor and the arm are pressed together; a pressing force determination unit that compares the force with determination values and determines whether or not the force is in a predetermined range; and a component concentration calculation unit that calculates a component concentration of the arm using the light intensity data that was output when the pressing force determination unit determined that the force is in the predetermined range.

## Description

### BACKGROUND

### 1. Technical Field

The present invention relates to a biological measurement apparatus and a biological measurement method.

### 2. Related Art

Review has been underway on methods for measuring the blood glucose level, blood pressure, and pulse of a person using near-infrared light and infrared light. For example, JP-A-11-188009 discloses a blood glucose level measurement apparatus that employs ATR (Attenuated Total Reflection). According to this technology, a glass plate having a reflective film provided on one surface is prepared. Then the surface of the glass plate not provided with the reflective film is brought into close contact with the skin of a biological object. Infrared light is emitted from one lateral surface of the glass plate. The infrared light is repeatedly reflected between the reflective film and the surface in close contact with the skin, and arrives at the other lateral surface of the glass plate. A sensor detects the light intensity of the infrared light that arrived at the other lateral surface.

The detected infrared light is spectrally separated to obtain spectral data. The skin of the biological object includes capillaries, and some of the infrared light emitted onto the surface of the skin is absorbed by the capillaries. There is a correlation between blood components and infrared light absorbed at specific wavelengths. Accordingly, there is a correlation between the concentration of blood components in the test biological object and light intensities detected at specific wavelengths. This makes it possible for the concentration of glucose in the blood, for example, to be detected using ATR.

JP-A-2000-254105 discloses a measurement apparatus in which an optical sensor is provided on a belt that is to be wrapped around an arm. According to this technology, the belt is provided on the measurement apparatus main body. The measurement apparatus is fixed by wrapping the belt around a person's arm. Also, the optical sensor is provided on the surface of the belt that faces the arm. The wrapped length of the belt around the arm is adjusted such that the optical sensor is used while being in close contact with the skin.

JP-A-11-188009 and JP-A-2000-254105 are examples of related art.

According to the ATR method disclosed in JP-A-11-188009, some of the infrared light is not absorbed by capillaries if the sensor unit is not in close contact with the skin of the biological object. Since the light intensity data does not reflect the state of the biological object in this case, it is not possible to detect an accurate blood component concentration. With the measurement apparatus disclosed in JP-A-2000-254105, a gap is formed between the optical sensor and the arm if the belt becomes loose. The light intensity detected by the optical sensor varies in this case. In view of this, there has been demand for a biological measurement apparatus that can precisely measure a component concentration of a biological object utilizing only light intensity data obtained when the detected light intensity data reflects the state of the biological object.

### SUMMARY

This invention has been achieved to address the above-described problems, and can be realized as modes or application examples described below.

### Application Example 1

A biological measurement apparatus according to this application example includes: an infrared sensor unit that irradiates a biological object with infrared light, receives infrared light that was reflected by the biological object, and outputs light intensity data; a holding unit that holds the infrared sensor unit such that the infrared sensor unit comes into contact with the biological object; a force sensor unit that detects force with which the infrared sensor unit and the biological object are pressed together; a determination unit that compares the force and a determination value, and determines whether or not the force is in a predetermined range; and a component concentration calculation unit that calculates a component concentration of the biological object using the light intensity data that was output when the determination unit determined that the force is in the predetermined range.

According to this application example, the biological measurement apparatus includes the holding unit, the infrared sensor unit, the force sensor unit, and the determination unit. The holding unit holds the infrared sensor unit such that the infrared sensor unit comes into contact with the biological object. The infrared sensor unit irradiates the biological object with infrared light. The infrared sensor unit then receives infrared light that was reflected by the biological object, and outputs light intensity data. The force sensor unit detects the force with which the infrared sensor unit and the biological object are pressed together. The determination unit then compares the force with the determination value and determines whether or not the force is in the predetermined range.

Infrared light is not absorbed by the biological object when the infrared sensor unit is separated from the biological object. Accordingly, the infrared sensor unit cannot precisely receive infrared light that reflects information regarding the biological object. Also, if the infrared sensor unit is pressed against the biological object with too much force, the blood vessels in the biological object are pressed, and blood flow in the vicinity of the surface is inhibited. Accordingly, the biological object is not in a state suitable to measurement, and the light intensity data received by the infrared sensor unit is data that does not correctly reflect the state of the biological object.

The determination unit determines that the infrared sensor unit is pressed against the biological object. The component concentration calculation unit then calculates a component concentration of the biological object utilizing data obtained when the light intensity data output by the infrared sensor unit reflected the state of the biological object, and not utilizing data obtained when the light intensity data did not reflect the state of the biological object. Accordingly, the biological measurement apparatus can measure the component concentration of the biological object with favorable precision by utilizing only the light intensity data obtained when the light intensity data reflected the state of the biological object.

### Application Example 2

It is preferable that the biological measurement apparatus according to the above application example further includes an infrared sensor control unit that controls driving of the infrared sensor unit, wherein the infrared sensor control unit drives the infrared sensor unit when the force is in the predetermined range.

According to this application example, the determination unit determines that the infrared sensor unit is pressed against the biological object. The infrared sensor control unit then drives the infrared sensor unit when the force applied to the biological object that was detected by the force sensor unit is in the predetermined range. Accordingly, the infrared sensor control unit drives the infrared sensor unit only when the infrared sensor unit can output appropriate light intensity data. This prevents wasting power for driving the infrared sensor unit, thus making it possible to save resources while driving the infrared sensor unit.

### Application Example 3

It is preferable that the biological measurement apparatus according to the above application example further includes a correction unit that corrects the light intensity data using data indicating the force, the light intensity data, and a table indicating a relationship between the force and a correction amount for the light intensity data.

According to this application example, the correction unit corrects the light intensity data using data indicating the force with which the infrared sensor unit and the biological object are pressed together, the light intensity data, and a table indicating the relationship between the force and correction amounts for the light intensity data. This makes it possible to detect the light intensity data with favorable precision even if the force with which the infrared sensor unit and the biological object are pressed together changes.

### Application Example 4

It is preferable that in the biological measurement apparatus according to the above application example, the determination unit compares forces output by a plurality of the force sensor units with a determination value, and determines whether or not the forces are in a predetermined range.

According to this application example, the biological measurement apparatus includes multiple force sensor units. The determination unit compares the forces output by the force sensor units with a determination value. The forces applied to the force sensor units by the biological object differ from each other if the infrared sensor unit is inclined relative to the biological object. Accordingly, it is also possible to detect a state in which the infrared sensor unit is inclined relative to the biological object. The infrared sensor unit can therefore detect the light intensity of the reflected infrared light when the infrared sensor unit is in a proper orientation relative to the biological object. As a result, information regarding the biological object can be detected with favorable precision.

### Application Example 5

It is preferable that in the biological measurement apparatus according to the above application example, the force sensor unit has a protrusion portion, and the protrusion portion protrudes from the infrared sensor unit toward the biological object.

According to this application example, the force sensor unit has a protrusion portion that protrudes toward the biological object. Accordingly, the protrusion portion of the force sensor unit can come into contact with the biological object even if the infrared sensor unit and the biological object are separated from each other. The force sensor unit can therefore reliably detect whether or not the infrared sensor unit and the biological object are in contact with each other.

### Application Example 6

It is preferable that in the biological measurement apparatus according to the above application example, the protrusion portion has an elastic body.

According to this application example, the protrusion portion has an elastic body. Accordingly, it is possible to suppress the case where the biological object becomes hypersensitive to the feeling of contact with the protrusion portion.

### Application Example 7

A biological measurement method according to this application example includes: a step of pressing an infrared sensor unit against a biological object; a step of detecting force with which the infrared sensor unit and the biological object are pressed together; a step of driving the infrared sensor unit when the force is in a predetermined range; and a step of, with the infrared sensor unit, irradiating the biological object with infrared light, receiving infrared light that was reflected by the biological object, and outputting light intensity data.

According to this application example, the infrared sensor unit is pressed against the biological object. The infrared sensor unit then irradiates the biological object with infrared light, receives infrared light that was reflected by the biological object, and outputs light intensity data. A blood glucose level is then calculated using the light intensity data. The force with which the infrared sensor unit and the biological object are pressed together is detected, and the infrared sensor unit is driven when the force is in the predetermined range. Accordingly, the infrared sensor unit detects light intensity data regarding the reflected infrared light only when the infrared sensor unit can detect the light intensity data with favorable precision. As a result, the light intensity of infrared light reflected by the biological object can be detected with favorable precision.

### Application Example 8

The biological measurement method according to the above application example further includes a step of correcting the light intensity data using data indicating the force, the light intensity data, and a table indicating a relationship between the force and a correction amount for the light intensity data.

According to this application example, the correction unit corrects the light intensity data using data indicating the force with which the infrared sensor unit and the biological object are pressed together, the light intensity data, and a table indicating the relationship between the force and correction amounts for the light intensity data. This makes it possible to detect the light intensity data with favorable precision even if the force with which the infrared sensor unit and the biological object are pressed together changes.

### BRIEF DESCRIPTION OF THE DRAWINGS

The invention will be described with reference to the accompanying drawings, wherein like numbers reference like elements.
Fig. 1A is an illustrative perspective view of a configuration of a biological measurement apparatus according to a first embodiment, Fig. 1B is a schematic front view of the configuration of the biological measurement apparatus according to the first embodiment, and Fig. 1C is a schematic side view of the configuration of the biological measurement apparatus according to the first embodiment.
Fig. 2A is an enlarged schematic side view of a relevant portion of a main body unit of the biological measurement apparatus, Fig. 2B is a schematic view of a structure of a relevant portion of an infrared sensor, and Fig. 2C is an enlarged schematic view of a structure of a relevant portion of a force sensor.
Fig. 3 is an electrical control block diagram of the biological measurement apparatus.
Fig. 4 is a flowchart of a biological measurement method.
Figs. 5A to 5D are schematic diagrams for describing the biological measurement method.
Figs. 6A to 6C are schematic diagrams for describing the biological measurement method.
Fig. 7 is an enlarged schematic view of a structure of a relevant portion of a force sensor according to a second embodiment.
Fig. 8A is an enlarged schematic view of a structure of a relevant portion of a main body unit according to a third embodiment. Figs. 8B to 8D are schematic diagrams for describing a biological measurement method according to the third embodiment.
Figs. 9A to 9C are schematic front views of arrangements of force sensors relative to an infrared sensor.

### DESCRIPTION OF EXEMPLARY EMBODIMENTS

Characteristic examples of a biological measurement apparatus and a biological measurement method that non-invasively performs blood glucose level measurement using the biological measurement apparatus will be described in the following embodiments with reference to figures. Embodiments will be described below with reference to the drawings. Note that the members in the drawings are shown at recognizable sizes in the drawings, and the members are illustrated at different scales.

### First Embodiment

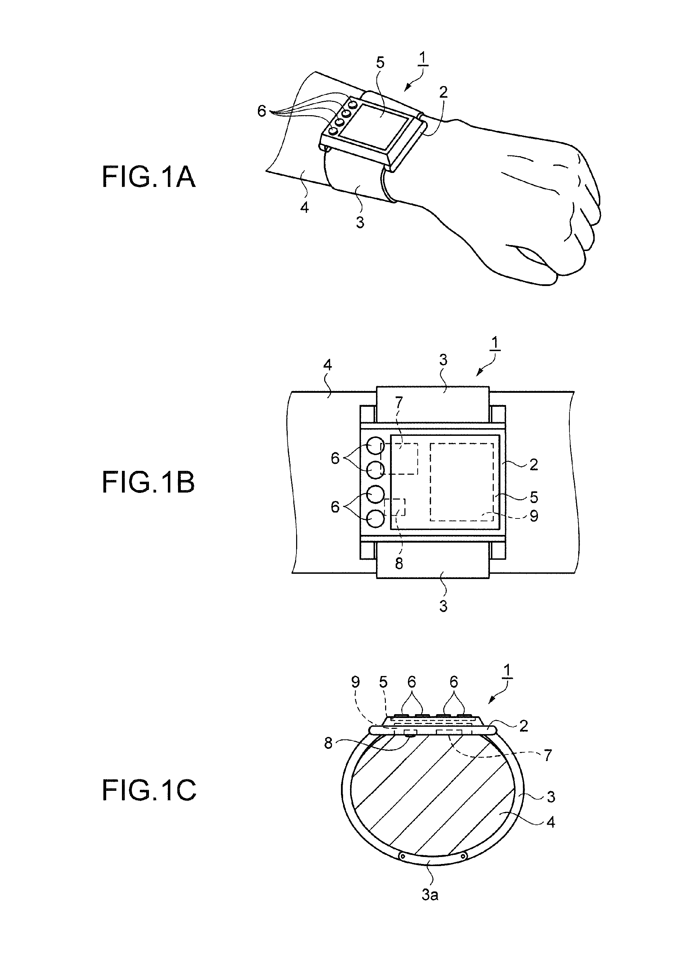

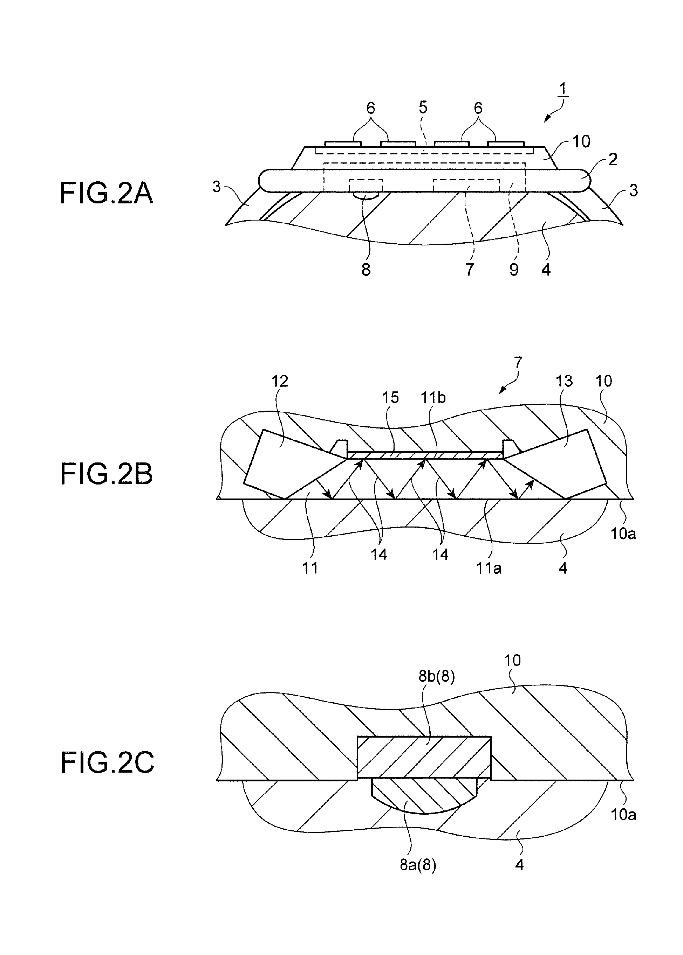

A biological measurement apparatus of a first embodiment will be described below with reference to Figs. 1A to 3. Fig. 1A is an illustrative perspective view of the configuration of the biological measurement apparatus, and Fig. 1B is a schematic front view of the configuration of the biological measurement apparatus. Fig. 1C is a schematic side view of the configuration of the biological measurement apparatus. Fig. 2A is an enlarged schematic side view of a relevant portion of a main body unit of the biological measurement apparatus.

As shown in Figs. 1A to 1C and Fig. 2A, a biological measurement apparatus 1 includes a main body unit 2 and a belt unit 3 provided on the main body unit 2. The main body unit 2 and the belt unit 3 form a tubular shape. An arm 4, which is a person's biological object, is inserted into the formed tube. In other words, the biological measurement apparatus 1 is used while being attached to the arm 4 like a watch.

A display apparatus 5 and an input apparatus 6 are provided on the surface of the main body unit 2 on the side opposite to the arm 4. The display apparatus 5 is a portion that displays information such as the measured blood glucose level. A liquid crystal display apparatus, an organic EL (Electro Luminescence) apparatus, or the like can be used as the display apparatus 5. The input apparatus 6 is constituted by switches and the like, and an operator can configure various settings by operating the input apparatus 6.

An infrared sensor 7 and a force sensor 8, which is a force sensor unit, are provided on the surface of the main body unit 2 that faces the arm 4. A control unit 9 is provided inside the main body unit 2. The control unit 9 controls the display apparatus 5, the input apparatus 6, the infrared sensor 7, and the force sensor 8. The main body unit 2 also includes a battery and a buzzer (neither of which are shown).

The belt unit 3 is provided with an adjustment unit 3a. The adjustment unit 3a enables adjustment of the length of the belt unit 3. The operator operates the adjustment unit 3a after attaching the biological measurement apparatus 1 to the arm 4. The length of the belt unit 3 is adjusted such that the infrared sensor 7 and the force sensor 8 come into contact with the arm 4. The belt unit 3 is made of metal, and is constructed by linking plate springs together. The belt unit 3 can thus expand and contract. A holding unit 10 is constituted by the main body unit 2 and the belt unit 3. The infrared sensor 7 and the force sensor 8 are held by the holding unit 10 so as to come into contact with the arm 4.

Fig. 2B is a schematic view of the structure of a relevant portion of the infrared sensor. As shown in Fig. 2B, the infrared sensor 7 includes a plate-shaped prism 11. The prism 11 has a pair of inclined opposing lateral surfaces. A light emitting apparatus 12 is provided on one of the inclined surfaces, and a light receiving apparatus 13 is provided on the other inclined surface. The prism 11 need only be made of a material that transmits infrared light, examples of which include ZnSe, Ge, and Si.

The light emitting apparatus 12 includes an LED (Light Emitting Diode) or the like, and the light emitting apparatus 12 emits infrared light 14 toward the interior of the prism 11. The light receiving apparatus 13 includes a spectrometer and a photodiode. The light receiving apparatus 13 separates the infrared light 14 into predetermined wavelengths, and converts the light intensities of the various wavelengths into electrical signals. The spectrometer is constituted by the combination of a diffraction grating, a concave mirror, and a slit. The spectrometer performs spectral separation by rotating the mirror such that light of a predetermined wavelength passes through the slit. The infrared light 14 that passes through the slit is converted into an electrical signal by the photodiode.

The surface of the prism 11 that comes into contact with the arm 4 is a first surface 11a, and the surface that opposes the first surface 11a is a second surface 11b. The first surface 11a and the second surface 11b are arranged parallel to each other. A reflective film 15 is provided on the second surface 11b. The reflective film 15 is preferably made of a metal that has a high reflectance, such as silver. The infrared light 14 emitted from the light emitting apparatus 12 travels toward the first surface 11a. The infrared light 14 then illuminates the surface of the arm 4 at the first surface 11a, and is reflected by the surface of the arm 4. The reflected infrared light 14 travels toward the reflective film 15, and is reflected at the reflective film 15. In this way, the infrared light 14 travels toward the light receiving apparatus 13 while being repeatedly reflected between the surface of the arm 4 at the first surface 11a and the second surface 11b.

Each time the infrared light 14 is reflected at the first surface 11a, a portion of the infrared light 14 with a predetermined wavelength is absorbed by capillaries in the arm 4. The light intensity of the infrared light 14 at a predetermined wavelength thus decreases. The infrared light 14 that arrives at the light receiving apparatus 13 is spectrally separated and converted into an electrical signal. The electrical signal is a signal that corresponds to the light intensity data. In other words, the infrared sensor 7 irradiates the arm 4 with infrared light 14, receives infrared light 14 that was reflected by the arm 4, and outputs light intensity data.

Considering the surface of the holding unit 10 that faces the arm 4 to be a contact surface 10a, the contact surface 10a is arranged in approximately the same plane as the first surface 11a. Accordingly, the first surface 11a reliably comes into contact with the arm 4.

Fig. 2C is an enlarged schematic view of the structure of a relevant portion of the force sensor. As shown in Fig. 2C, the force sensor 8 includes a sensor main body unit 8b and a pressing protrusion 8a, which is a protrusion portion. A piezoelectric element is built into the sensor main body unit 8b, and the piezoelectric element is sandwiched between the sensor main body unit 8b and the pressing protrusion 8a in this structure. When the pressing protrusion 8a is pressed toward the sensor main body unit 8b, force is applied to the piezoelectric element. The piezoelectric element then outputs the differential value of voltage generated in accordance with the force. Accordingly, the pressure force applied to the force sensor 8 can be detected using the voltage output by the piezoelectric element.

The infrared sensor 7 and the force sensor 8 are held by the holding unit 10 and come into contact with the arm 4. Since the infrared sensor 7 and the force sensor 8 are provided close to each other, the pressure force applied to the infrared sensor 7 by the arm 4 and the pressure force applied to the force sensor 8 by the arm 4 are approximately the same pressure force. By detecting the pressing force applied to the force sensor 8, it is possible to detect the pressing force applied to the infrared sensor 7. The force sensor 8 can thus detect the force with which the infrared sensor 7 and the arm 4 are pressed together.

The pressing protrusion 8a protrudes from the contact surface 10a of the holding unit 10, toward the arm 4. The contact surface 10a is approximately flush with the first surface 11a of the prism 11. The pressing protrusion 8a therefore protrudes from the infrared sensor 7 toward the arm 4. Accordingly, the pressing protrusion 8a can come into contact with the arm 4 even when the infrared sensor 7 and the arm 4 are separated from each other. The force sensor 8 can thus reliably detect whether or not the infrared sensor 7 and the arm 4 are in contact with each other.

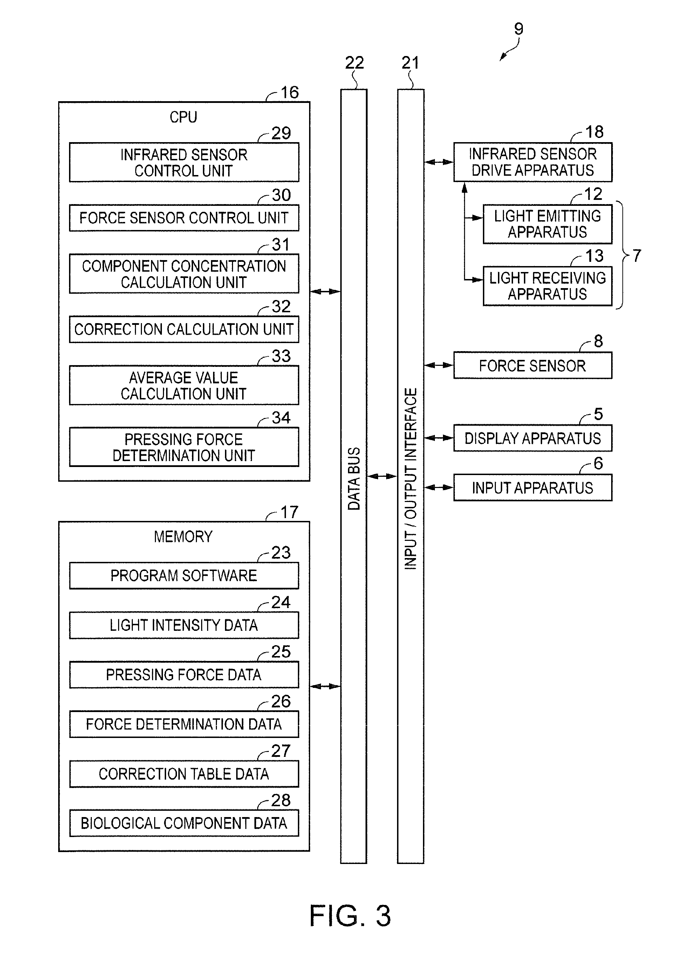

Fig. 3 is an electrical control block diagram of the biological measurement apparatus. As shown in Fig. 3, the control unit 9 of the biological measurement apparatus 1 has a CPU 16 (operation processing unit) that performs various types of operation processing as a processor, and a memory 17 that stores various types of information.

An infrared sensor drive apparatus 18, the force sensor 8, the input apparatus 6, and the display apparatus 5 are connected to the CPU 16 via an input/output interface 21 and a data bus 22. The infrared sensor drive apparatus 18 is an apparatus that drives the light emitting apparatus 12 and the light receiving apparatus 13. The infrared sensor drive apparatus 18 drives the light emitting apparatus 12 so as to emit light upon receiving an instruction signal from the CPU 16. The infrared sensor drive apparatus 18 then drives the light receiving apparatus 13 and receives light intensities for various wavelengths output from the light receiving apparatus 13. The infrared sensor drive apparatus 18 outputs light intensity data including the measured spectrums and the like to the CPU 16.

In addition to switches, the input apparatus 6 includes an interface for receiving various types of setting information from an external device. Data and the like that are to be used when extracting a biological component can thus being received by the biological measurement apparatus 1 from an external apparatus via the input apparatus 6.

The memory 17 is a concept including a semiconductor memory such as a RAM or a ROM, and an external storage apparatus such as an external hard disk or a CD-ROM. In terms of functionality, a storage region is set for storing program software 23 that describes a control procedure for operations in the biological measurement apparatus 1. A storage region is also set for the storage of light intensity data 24, which is spectrum data, by the infrared sensor 7. Additionally, a storage region is set for storing pressing force data 25, which is data that is output by the force sensor 8 and indicates the force with which the infrared sensor 7 and the arm 4 are pressed together.

Furthermore, a storage region is set for storing force determination data 26, which is data used when the CPU 16 makes a determination using the pressing force data 25. Moreover, a storage region is set for storing correction table data 27, which is data used when the CPU 16 corrects the light intensity data 24 using the pressing force data 25. The correction table data 27 is data that indicates the relationship between pressing force that the arm 4 applies to the infrared sensor 7 and correction amounts for the light intensity data 24. Moreover, a storage region is set for storing biological component data 28, which is measured component concentration data and data used when the CPU 16 calculates a blood component concentration. Moreover, a storage region that functions as a work area for the CPU 16, a temporary file area, and the like are set, and various other types of storage regions are set.

In accordance with the program software 23 stored in the memory 17, the CPU 16 performs control for detecting a blood component using data output by the infrared sensor 7. As a specific function achieving unit, the CPU 16 has an infrared sensor control unit 29 that instructs the infrared sensor drive apparatus 18 to drive the infrared sensor 7. Furthermore, the CPU 16 has a force sensor control unit 30 that instructs the force sensor 8 to detect the pressing force between the infrared sensor 7 and the arm 4. Moreover, the CPU 16 has a component concentration calculation unit 31 that calculates a blood component concentration using the light intensity data 24 detected by the infrared sensor 7.

The CPU 16 further has a correction calculation unit 32 as a correction unit that performs calculation for correcting the light intensity data 24 using the pressing force data 25. The CPU 16 further has an average value calculation unit 33 that calculates an average value for the biological component data 28 and the blood component concentration. The CPU 16 further has a pressing force determination unit 34 as a determination unit that determines whether or not the light intensity data 24 is to be used, based on the pressing force data 25 and the force determination data 26.

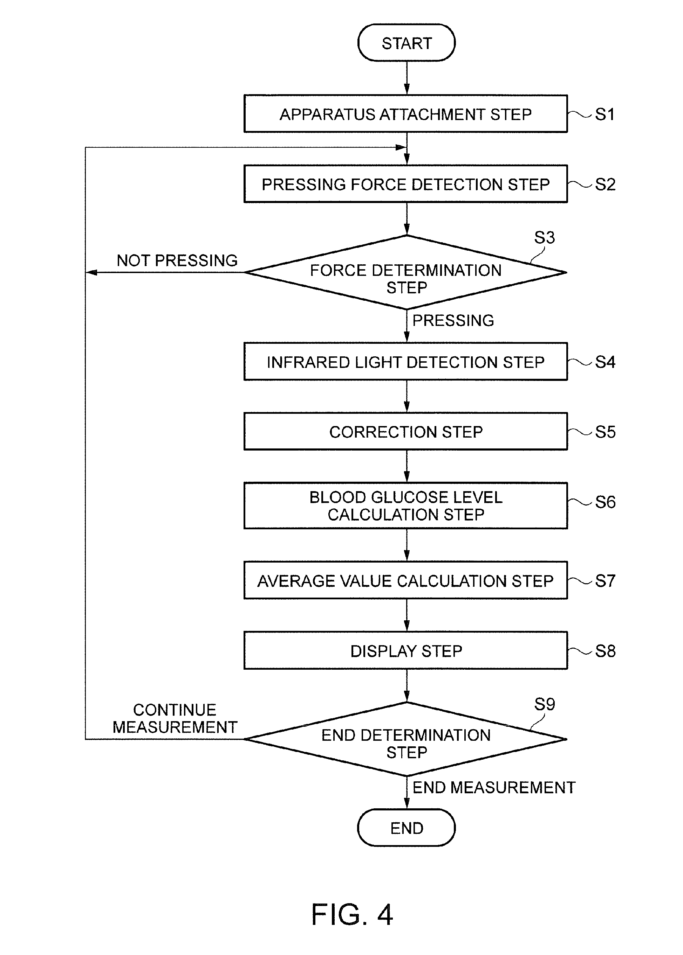

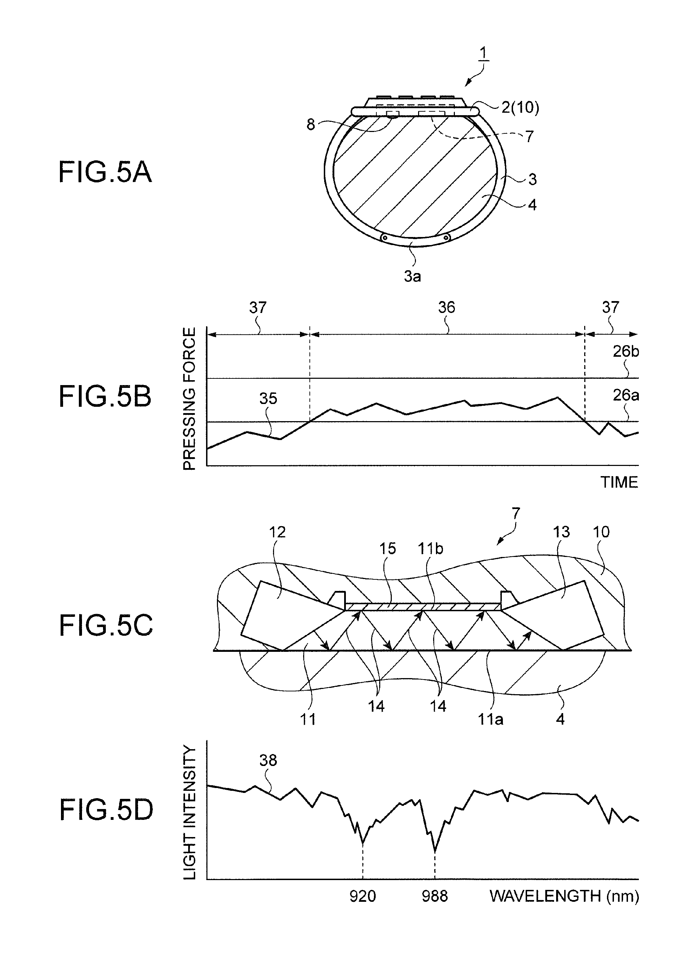

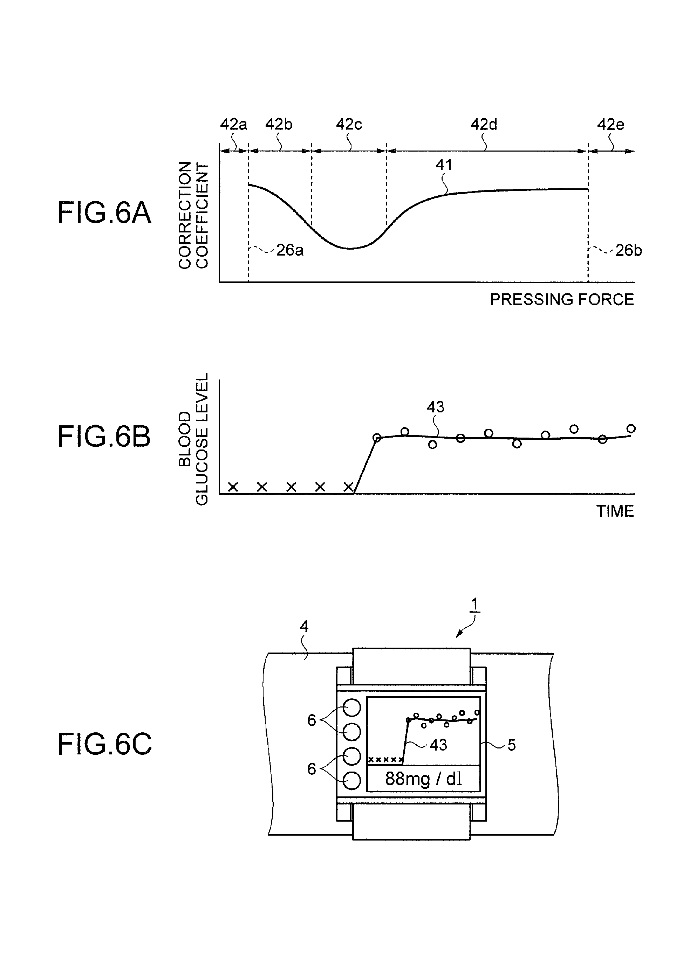

Next, a biological measurement method for calculating a blood component concentration using the above-described biological measurement apparatus 1 will be described with reference to Figs. 4 to 6C. Fig. 4 is a flowchart of the biological measurement method, and Figs. 5A to 5D and 6A to 6C are schematic diagrams for describing the biological measurement method.

In the flowchart of Fig. 4, step S1 corresponds to an apparatus attachment step, which is a step in which the biological measurement apparatus 1 is attached to the arm 4. In this step, the biological measurement apparatus 1 is attached such that the infrared sensor 7 presses against the arm 4. Next, the procedure moves to step S2. Step S2 corresponds to a pressing force detection step. This step is a step in which the force sensor 8 detects the pressing force, which is the force with which the infrared sensor 7 and the arm 4 are pressed together. Next, the procedure moves to step S3. Step S3 corresponds to a force determination step, which is a step in which it is determined whether or not the detected pressing force is in a determination value range. If it is determined that the pressing force is in the determination value range, and that the arm 4 and the infrared sensor 7 are pressed together, the procedure moves to step S4. If it is determined that the pressing force is in the determination value range, and that the arm 4 and the infrared sensor 7 are not pressed together, the procedure moves to step S2.

Step S4 corresponds to an infrared light detection step. In this step, the infrared sensor drive apparatus 18 drives the infrared sensor 7. The infrared sensor 7 thus irradiates the arm 4 with the infrared light 14. In this step, the infrared light 14 that is reflected by the arm 4 is received by the infrared sensor 7, which outputs the light intensity data 24 indicating the spectrum of the reflected light. Next, the procedure moves to step S5. Step S5 corresponds to a correction step. This step is a step in which the light intensity data 24 is corrected using the pressing force data 25 and the correction table data 27. Next, the procedure moves to step S6. Step S6 corresponds to a blood glucose level calculation step. This step is a step in which blood glucose levels are calculated using the corrected light intensity data 24. Next, the procedure moves to step S7.

Step S7 corresponds to an average value calculation step. This step is a step in which the average value of the blood glucose levels is calculated. Next, the procedure moves to step S8. Step S8 corresponds to a display step. This step is a step in which the calculated average value of the blood glucose levels is displayed on the display apparatus 5. Next, the procedure moves to step S9. Step S9 corresponds to an end determination step, which is a step in which it is determined whether measurement is to be ended or continued. If measurement is to be continued, the procedure moves to step S2. If measurement is to be ended, the biological object measurement step is ended.

Next, details of the biological measurement method will be described in accordance with the steps shown in Fig. 4, with reference to Figs. 5A to 5D and Figs. 6A to 6C. Fig. 5A is a diagram corresponding to the apparatus attachment step of step S1. As shown in Fig. 5A, the operator attaches the biological measurement apparatus 1 to the arm 4. The operator then operates the adjustment unit 3a so as to adjust the length of the belt unit 3. The operator then presses the infrared sensor 7 and the force sensor 8 against the arm 4 so as to bring them into close contact with the arm 4. At this time, the operator adjusts the length of the belt unit 3 such that the belt unit 3 does not constrict the arm 4, and such that blood flows freely through the capillaries of the arm 4.

Fig. 5B is a diagram corresponding to the pressing force detection step of step S2 and the force determination step of step S3. In Fig. 5B, the vertical axis indicates the pressing force detected by the force sensor 8, and the force increases from bottom to top in the figure. The horizontal axis indicates the elapse of time, and time moves from left to right in the figure. In step S2, the force sensor control unit 30 outputs an instruction for causing the force sensor 8 to detect the pressing force. The force sensor 8 stores the pressing force applied by the arm 4 in the memory 17 as the pressing force data 25. A force change line 35 indicates change in the pressing force detected by the force sensor 8.

The force determination data 26 is stored in the memory 17. The force determination data 26 includes a lower determination value 26a and an upper determination value 26b. The pressing force determination unit 34 compares the pressing force with the lower determination value 26a. If the pressing force is smaller than the lower determination value 26a, it is determined that the pressing force is insufficient. The pressing force determination unit 34 also compares the pressing force with the upper determination value 26b. If the pressing force is larger than the upper determination value 26b, it is determined that the pressing force is excessive.

If the pressing force is greater than or equal to the lower determination value 26a, and less than or equal to the upper determination value 26b, the pressing force determination unit 34 determines that the pressing force is appropriate. An appropriate section 36 in the force change line 35 is the section in which the pressing force is appropriate. Also, inappropriate sections 37 in the force change line 35 are sections in which the pressing force is insufficient or excessive.

In the inappropriate sections 37, the message "Detection Error" is displayed on the display apparatus 5. Furthermore, a sound is emitted by the buzzer to prompt the operator to perform adjustment. Step S2 and step S3 are then repeated. In the appropriate section 36 in which the pressing force is appropriate, the procedure moves to step S4.

Figs. 5C and 5D are diagrams corresponding to the infrared light detection step of step S4. In step S4, the infrared sensor control unit 29 outputs an instruction signal for driving the infrared sensor 7 to the infrared sensor drive apparatus 18. The infrared sensor control unit 29 drives the infrared sensor 7 only when the pressing force is appropriate. As shown in Fig. 5C, the infrared sensor drive apparatus 18 drives the light emitting apparatus 12 to emit light, and the light emitting apparatus 12 emits the infrared light 14 into the prism 11. Inside the prism 11, the infrared light 14 travels toward the light receiving apparatus 13 while being repeatedly reflected between the second surface 11b and the surface of the arm 4 that is in close contact with the first surface 11a. The infrared light 14 illuminates the surface of the arm 4 at the first surface 11a. Infrared light 14 at some wavelengths is absorbed by the blood in the capillaries of the arm 4.

The light receiving apparatus 13 detects a light intensity for each wavelength. As a result, the spectrum of the infrared light 14 is output as shown in Fig. 5D. In this figure, the vertical axis indicates the light intensity, and the light intensity increases from bottom to top in the figure. The horizontal axis indicates the wavelength, and the wavelength increases in length from left to right in the figure. A light intensity distribution line 38 indicates the light intensity of the infrared light 14 at the respective wavelengths. In the light intensity distribution line 38, the light intensity is small at the 920 nm and 988 nm wavelength locations. The infrared light 14 is absorbed by the blood in the capillaries at these wavelengths. Also, more of the infrared light 14 is absorbed the higher the blood glucose concentration is.

Fig. 6A is a diagram corresponding to the correction step of step S5. In step S5, the correction calculation unit 32 corrects the light intensity distribution line 38. The correction table data 27 is stored in the memory 17. A correction curve 41 shown in Fig. 6A is a curve indicating a table in the correction table data 27. The correction table data 27 indicates the relationship between the pressing force detected by the force sensor 8 and the correction amount with which the infrared sensor 7 performs correction. The correction curve 41 is a curve set based on the relationship between the pressing force and the light intensity detected by the infrared sensor 7. In this figure, the vertical axis indicates the correction coefficient, and the correction coefficient increases from bottom to top in the figure. The horizontal axis indicates the pressing force, and the pressing force increases from left to right in the figure. In this relationship, the correction amount increases as the correction coefficient increases.

The correction curve 41 will be described divided into sections, namely a first section 42a, a second section 42b, a third section 42c, a fourth section 42d, and a fifth section 42e. The first section 42a is a section in which the pressing force is smaller than the lower determination value 26a, and is a section in which the contact between the arm 4 and the first surface 11a of the prism 11 is unstable. Accordingly, the infrared light detection step of step S4 is not performed, and the correction curve 41 is not set.

The second section 42b is a section in which there is a possibility of the infrared sensor 7 detecting the absorption of the infrared light 14 by the blood in the capillaries, but the pressing force between the infrared sensor 7 and the arm 4 is low. The second section 42b is a section in which the absorption of the infrared light 14 by the blood in the capillary increases as the pressing force increases. In this section, the correction coefficient increases as the pressing force decreases.

The third section 42c is a section in which the pressing force is appropriate, and the infrared sensor 7 can appropriately detect the absorption of the infrared light 14 by the blood in the capillaries. In this section, the extent of the correction according to the light intensity distribution line 38 is lowered.

The fourth section 42d is a section in which there is a possibility of the infrared sensor 7 detecting the absorption of the infrared light 14 by the blood in the capillaries, but the amount of blood is low due to the capillaries being pressed. The stronger the infrared sensor 7 is pressed against the arm 4, the lower the amount of blood is, and the lower the absorption of the infrared light 14 is. Accordingly, in this section, the correction coefficient increases as the pressing force increases.

The fifth section 42e is a section in which the ability of the infrared sensor 7 to detect the absorption of the infrared light 14 by the blood in the capillaries is unstable. Accordingly, the infrared light detection step of step S4 is not performed, and the correction curve 41 is not set.

In step S5, the correction calculation unit 32 calculates a correction coefficient based on the pressing force and the correction curve 41, and multiplies the light intensity distribution line 38 by the correction coefficient. If the operator moves the arm 4, the relative positions of the arm 4 and the biological measurement apparatus 1 change. The pressing force between the infrared sensor 7 and the arm 4 also changes at this time. The correction calculation unit 32 thus corrects the light intensity distribution line 38 using the pressing force and the correction curve 41. Accordingly, it is possible to obtain a favorably precise light intensity distribution line 38 even if the pressing force changes.

In the blood glucose level calculation step of step S6, the component concentration calculation unit 31 calculates the blood glucose level from the light intensity distribution line 38. A light intensity/blood glucose level correlation table showing the relationship between the blood glucose level and the light intensity at predetermined wavelengths is stored in the biological component data 28. The component concentration calculation unit 31 calculates the blood glucose level using the light intensity/blood glucose level correlation table and the light intensity distribution line 38. The blood glucose level obtained here indicates the amount of glucose in the blood. Note that the blood glucose level may be calculated with even higher precision by applying PLS (Partial Least Squares) regression.

The blood glucose level calculation result is stored in the memory 17 as the biological component data 28. Then, after measurement is ended, it is possible to transmit the blood glucose level data from the biological measurement apparatus 1 to an external device such as a personal computer. Accordingly, the change in the blood glucose level can be analyzed by a program installed in the personal computer.

Fig. 6B is a diagram corresponding to the average value calculation step of step S7. In Fig. 6B, the vertical axis indicates the measured blood glucose level, and the blood glucose level increases from bottom to top in the figure. The horizontal axis indicates the elapse of measurement time, and time moves from left to right in the figure. Also, the plotted cross marks indicate that measurement was not performed, and the plotted circle marks indicate examples of the measured blood glucose level. A blood glucose level change line 43 indicates the result of calculating the simple moving average for the measurement value.

The average value calculation unit 33 calculates the simple moving average using only data regarding blood glucose levels that were measured, that is to say, without using data obtained when measurement was not performed. Although there is no particular limitation on the number of data points, the maximum number of data points is 10 for example in this embodiment. If the number of data points is lower than the maximum number of data points, the average value of the measured data is calculated. If the number of data points is greater than or equal to 10 data points, the average value is calculated for the 10 data points whose measurement times are the closest to the current time. Accordingly, the average value is updated when new data is measured.

Fig. 6C is a diagram corresponding to the display step of step S8. As shown in Fig. 6C, in step S8, the measurement results are displayed on the display apparatus 5. The calculated blood glucose level plots and the blood glucose level change line 43 are displayed on the display apparatus 5. Furthermore, the most recently obtained average value is displayed.

In the end determination step of step S9, the operator determines whether blood glucose level measurement is to be continued or ended. In the case of ending blood glucose level measurement, this is instructed by pressing a switch of the input apparatus 6. The CPU 16 receives a signal from the input apparatus 6 and stops the program for performing measurement. According to the above step, the biological measurement procedure is ended.

As described above, this embodiment obtains effects such as the following.
(1) According to this embodiment, the pressing force determination unit 34 determines that the infrared sensor 7 is pressed against the arm 4. The component concentration calculation unit 31 then calculates the blood glucose level of the blood utilizing data obtained when the light intensity data output by the infrared sensor 7 reflects the state of the blood, and without utilizing data obtained when the light intensity data does not reflect the state of the blood. Accordingly, the biological measurement apparatus 1 can measure the blood glucose level with favorable precision by utilizing only the light intensity data obtained when the light intensity data reflects the state of the biological object.
(2) According to this embodiment, the pressing force determination unit 34 determines that the infrared sensor 7 is pressed against the arm 4. The infrared sensor control unit 29 then drives the infrared sensor 7 when the force with which the infrared sensor 7 presses the arm 4 is between the lower determination value 26a and the upper determination value 26b. Accordingly, the infrared sensor control unit 29 drives the infrared sensor 7 only when the infrared sensor 7 can output appropriate light intensity data. This prevents wasting power for driving the infrared sensor 7, thus making it possible to save resources while driving the infrared sensor 7.
(3) According to this embodiment, the correction calculation unit 32 corrects the light intensity data using data indicating the pressing force with which the infrared sensor 7 and the arm 4 are pressed together, the light intensity data, and the table indicating the relationship between the pressing force and the light intensity data correction amount. This makes it possible to detect the light intensity data with favorable precision even if the force with which the infrared sensor 7 and the arm 4 are pressed together changes.
(4) According to this embodiment, the force sensor 8 has the pressing protrusion 8a that protrudes toward the arm 4. Accordingly, the pressing protrusion 8a of the force sensor 8 can come into contact with the arm 4 even when the infrared sensor 7 and the arm 4 are separated from each other. The force sensor 8 can thus reliably detect whether or not the infrared sensor 7 and the arm 4 are in contact with each other.

### Second Embodiment

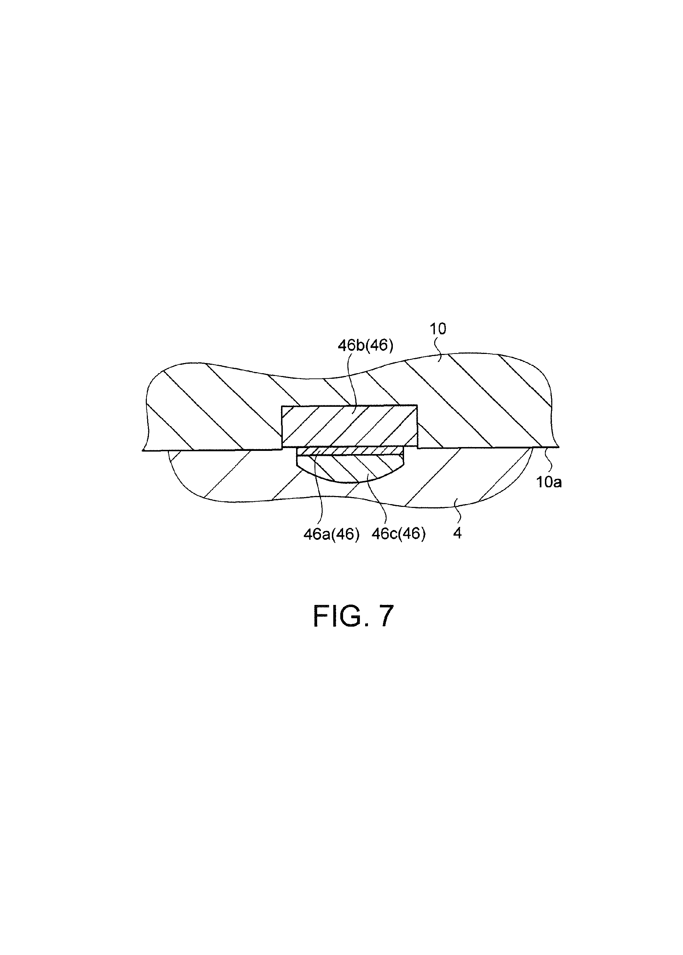

Next, another embodiment of the biological measurement apparatus will be described with reference to Fig. 7, which is an enlarged schematic view of the structure of a relevant portion of a force sensor.

This embodiment differs from the first embodiment in that a portion of the pressing protrusion 8a shown in Fig. 2C is an elastic body. Note that aspects that are the same as in the first embodiment will not be described.

In this embodiment, as shown in Fig. 7, a force sensor 46 has a sensor main body unit 46b, and a pressing protrusion 46a is provided on the arm 4 side of the sensor main body unit 46b. A piezoelectric element is built into the sensor main body unit 46b, and the piezoelectric element is sandwiched between the sensor main body unit 46b and the pressing protrusion 46a in this structure. A protrusion tip portion 46c, which is an elastic body, is provided on the arm 4 side of the pressing protrusion 46a. Pressing force that the arm 4 applies to the force sensor 46 is applied to the force sensor 46 via the protrusion tip portion 46c.

The material making up the protrusion tip portion 46c is an elastic body and has elasticity. There is no particular limitation on the material making up the protrusion tip portion 46c as long as it is an elastic body. Silicone rubber, synthetic rubber, natural rubber, as well as a metal coil spring or the like can be used. In this embodiment, silicone rubber is employed as the material making up the protrusion tip portion 46c.

As described above, this embodiment obtains effects such as the following.
(1) According to this embodiment, the protrusion tip portion 46c has an elastic body. Accordingly, it is possible to suppress the case where the arm 4 becomes hypersensitive to the feeling of contact with the protrusion tip portion 46c.

### Third Embodiment

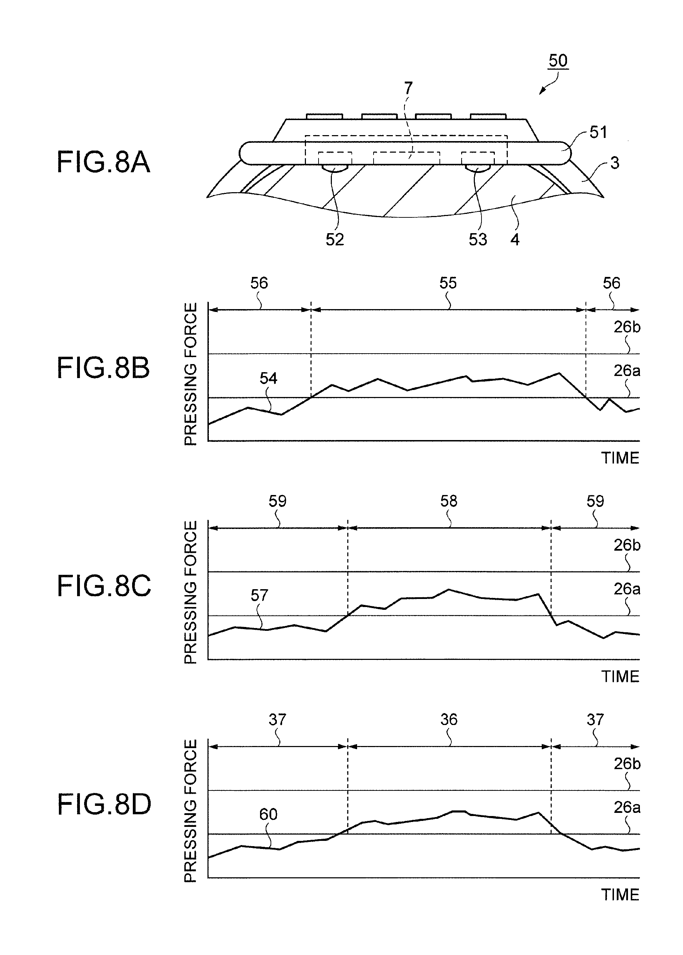

Next, another embodiment of the biological measurement apparatus will be described with reference to Figs. 8A to 8D. Fig. 8A is an enlarged schematic view of the structure of a relevant portion of the main body unit. Figs. 8B to 8D are schematic diagrams for describing the biological measurement method. This embodiment differs from the first embodiment in that multiple force sensors 8 are provided on the holding unit 10. Note that aspects that are the same as in the first embodiment will not be described.

In this embodiment, as shown in Fig. 8A, a biological measurement apparatus 50 includes a main body unit 51, and the infrared sensor 7 is provided at the center of the arm 4 side of the main body unit 51. Also, a first force sensor 52 and a second force sensor 53 are arranged on opposing sides of the infrared sensor 7.

Figs. 8B, 8C, and 8D are diagrams corresponding to the pressing force detection step of step S2 and the force determination step of step S3. In Fig. 8B, the vertical axis indicates the pressing force detected by the first force sensor 52, and the force increases from bottom to top in the figure. The horizontal axis indicates the elapse of time, and time moves from left to right in the figure. A first force change line 54 indicates an example of change in the pressing force detected by the first force sensor 52.

The pressing force determination unit 34 compares the pressing force detected by the first force sensor 52 with the lower determination value 26a. The pressing force determination unit 34 also compares the pressing force detected by the first force sensor 52 with the upper determination value 26b. A first appropriate section 55 in the first force change line 54 is the section in which the pressing force is appropriate. Also, first inappropriate sections 56 in the first force change line 54 are sections in which the pressing force is insufficient or excessive.

The horizontal axis and the vertical axis in Fig. 8C are the same as in Fig. 8B, and therefore will not be described. A second force change line 57 indicates an example of change in the pressing force detected by the second force sensor 53. The pressing force determination unit 34 compares the pressing force detected by the second force sensor 53 with the lower determination value 26a. The pressing force determination unit 34 also compares the pressing force detected by the second force sensor 53 with the upper determination value 26b. A second appropriate section 58 in the second force change line 57 is the section in which the pressing force is appropriate. Also, second inappropriate sections 59 in the second force change line 57 are sections in which the pressing force is insufficient or excessive.

As shown in Fig. 8D, the pressing force determination unit 34 considers the section that is included in both the first appropriate section 55 and the second appropriate section 58 to be the appropriate section 36. Also, the sections outside the appropriate section 36 are considered to be the inappropriate sections 37. In other words, if the pressing force detected by the first force sensor 52 and the pressing force detected by the second force sensor 53 are both greater than or equal to the lower determination value 26a and less than or equal to the upper determination value 26b, it is determined that the pressing force is appropriate. In the example of the first force change line 54 and the second force change line 57, the second appropriate section 58 is included in the first appropriate section 55, and therefore the appropriate section 36 is the same section as the second appropriate section 58.

The average value calculation unit 33 calculates a pressing force average value, which is the average value of the pressing force detected by the first force sensor 52 and the pressing force detected by the second force sensor 53. The horizontal axis and the vertical axis are the same as in Fig. 8B, and therefore will not be described. A pressing force average value change line 60 is a change line obtained by calculating the average of the first force change line 54 and the second force change line 57. In the correction step of step S5, the light intensity distribution line 38 is corrected using the pressing force average value change line 60 and the correction curve 41. In other words, the average value of the pressing force detected by the first force sensor 52 and the pressing force detected by the second force sensor 53 is applied as the pressing force that the arm 4 applies to the infrared sensor 7. Accordingly, the pressing force that the arm 4 applies to the infrared sensor 7 can be detected with favorable precision.

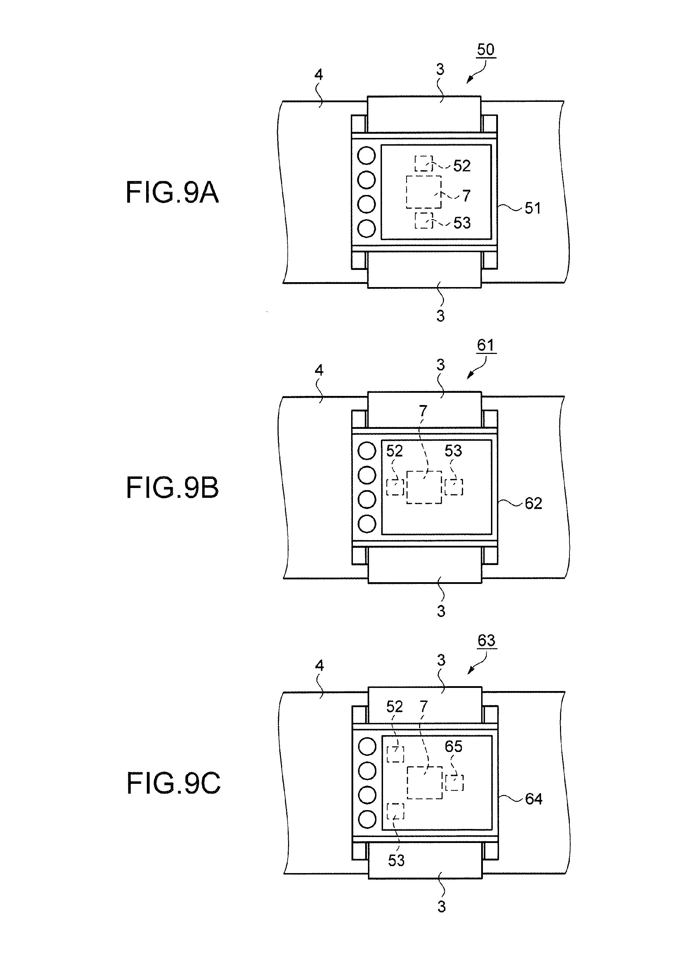

Figs. 9A, 9B, and 9C are schematic front views of arrangements of the force sensors relative to the infrared sensor. In Fig. 9A, in the biological measurement apparatus 50, the first force sensor 52 and the second force sensor 53 are provided on opposing sides of the infrared sensor 7 on the main body unit 51. The first force sensor 52, the infrared sensor 7, and the second force sensor 53 are arranged in a line extending in the direction orthogonal to the lengthwise direction of the arm 4. In this mode, it is possible to detect an inclined state of the main body unit 51 with the lengthwise direction of the arm 4 as the axis of rotation.

In Fig. 9B, in a biological measurement apparatus 61, the first force sensor 52 and the second force sensor 53 are provided on opposing sides of the infrared sensor 7 on a main body unit 62. The first force sensor 52, the infrared sensor 7, and the second force sensor 53 are arranged in a line extending in the lengthwise direction of the arm 4. In this mode, it is possible to detect an inclined state of the main body unit 62 with the direction orthogonal to the lengthwise direction of the arm 4 as the axis of rotation.

In Fig. 9C, in a biological measurement apparatus 63, the infrared sensor 7, the first force sensor 52, the second force sensor 53, and a third force sensor 65 are arranged on a main body unit 64. The first force sensor 52, the second force sensor 53, and the third force sensor 65 are arranged on a concentric circle centered about the infrared sensor 7. The angle formed by the first force sensor 52 and the second force sensor 53 with the infrared sensor 7 serving as the center is 120 degrees, and the angle formed by the first force sensor 52 and the third force sensor 65 with the infrared sensor 7 serving as the center is 120 degrees.

In this mode, using the first force sensor 52 and the second force sensor 53, it is possible to detect an inclined state of the main body unit 64 with the lengthwise direction of the arm 4 as the axis of rotation. Furthermore, using the first force sensor 52, the second force sensor 53, and the third force sensor 65, it is possible to detect an inclined state of the main body unit 64 with the direction orthogonal to the lengthwise direction of the arm 4 as the axis of rotation. Accordingly, it is possible to detect inclination of the main body unit 64 in any direction.

As described above, this embodiment obtains effects such as the following.
(1) According to this embodiment, the biological measurement apparatus 50 includes the first force sensor 52 and the second force sensor 53. Also, the pressing force determination unit 34 compares the pressing forces detected by the force sensors with the lower determination value 26a and the upper determination value 26b. Accordingly, it is also possible to detect a state in which the infrared sensor 7 is inclined relative to the arm 4. When the infrared sensor 7 is inclined, there are cases where one portion of the infrared sensor 7 is in close contact with the arm 4 while another portion is separated from the arm 4, and the infrared light 14 cannot be absorbed by the capillary with favorable precision. In this embodiment, the infrared sensor 7 can detect the light intensity of the reflected infrared light 14 when the infrared sensor 7 is in a proper orientation relative to the arm 4. As a result, the biological measurement apparatus 50, the biological measurement apparatus 61, and the biological measurement apparatus 63 can detect the blood glucose level of blood in the arm 4 with favorable precision.

Note that the embodiments of this invention are not limited to the embodiments described above, and a person with ordinary knowledge in this field can make various modifications and improvements within the technical idea of this invention. Some variations will be described below.

### Variation 1

In the first embodiment, the light receiving apparatus 13 has a function for performing spectral separation. There is no limitation to this, and the light emitting apparatus 12 may emit infrared light 14 of a predetermined wavelength. The light receiving apparatus 13 may then detect the light intensity without performing spectral separation. For example, the light emitting apparatus 12 may include multiple LEDs that emit different wavelengths of light. The operating LEDs may be switched. Furthermore, the wavelength may be shifted using a diffraction grating. Infrared light 14 of a predetermined wavelength may be emitted using various types of methods.

### Variation 2

In the first embodiment, the intensity distribution of the infrared light 14 relative to the wavelength of the light emitting apparatus 12 is not taken into consideration. The correction calculation unit 32 may correct the data indicating the light intensity distribution line 38 detected by the infrared sensor 7 in accordance with the intensity distribution of the infrared light 14 relative to the wavelength of the light emitting apparatus 12. This makes it possible to improve the precision of the blood component concentration.

### Variation 3

In the first embodiment, the light receiving apparatus 13 outputs data indicating the light intensity distribution line 38. The light receiving apparatus 13 may detect only the light intensity for a predetermined wavelength. By decreasing the number of wavelengths of infrared light 14 that are to be detected, the light receiving apparatus 13 can output the light intensity for a predetermined wavelength in a short period of time.

### Variation 4

In the first embodiment, the control unit 9 is accommodated inside the main body unit 2. If the control unit 9 cannot be fully accommodated inside the main body unit 2, part or all of the control unit 9 may be accommodated in an electrical apparatus separate from the main body unit 2. In this case, the electrical apparatus and the main body unit 2 may communicate via a wired or wireless connection. Accordingly, the biological measurement apparatus 1 can be made easier to manufacture.

### Variation 5

In the first embodiment, the blood glucose level is measured. Another component concentration may be measured by changing the analyzed wavelength of infrared light 14. Additionally, the pulse and blood pressure may be measured by irradiating multiple locations with the infrared light 14 and detecting the pulse wave of the blood flow.

### Variation 6

In the first embodiment, the infrared sensor control unit 29 drives the infrared sensor 7 in only the appropriate section 36. The infrared sensor 7 may be driven at all times. In this case, the light intensity data 24 may be applied in only the appropriate section 36. This makes it possible to make the control of the infrared sensor 7 easier.

### Variation 7

In the first embodiment, the correction calculation unit 32 corrects the light intensity distribution line 38. Correction may be omitted if the influence of correction is small. This makes it possible to reduce the data size of the program software 23. Note that the content of Variations 1 to 7 may be applied to the second embodiment and the third embodiment as well.

### Variation 8

In the first embodiment, the average value calculation unit 33 calculates a simple moving average. The average value calculation unit 33 may calculate a simple average. For example, the predetermined number of data points may be set to 20, and an average value may be calculated each time measurement values corresponding to the predetermined number of data points have been stored. Calculation may be performed according to demand.

## Claims

1. A biological measurement apparatus comprising:
an infrared sensor unit that irradiates a biological object with infrared light, receives infrared light that was reflected by the biological object, and outputs light intensity data;
a holding unit that holds the infrared sensor unit such that the infrared sensor unit comes into contact with the biological object;
a force sensor unit that detects force with which the infrared sensor unit and the biological object are pressed together;
a determination unit that compares the force and a determination value, and determines whether or not the force is in a predetermined range; and
a component concentration calculation unit that calculates a component concentration of the biological object using the light intensity data that was output when the determination unit determined that the force is in the predetermined range.

2. The biological measurement apparatus according to claim 1, further comprising an infrared sensor control unit that controls driving of the infrared sensor unit,
wherein the infrared sensor control unit drives the infrared sensor unit when the force is in the predetermined range.

3. The biological measurement apparatus according to claim 1 or claim 2, further comprising a correction unit that corrects the light intensity data using data indicating the force, the light intensity data, and a table indicating a relationship between the force and a correction amount for the light intensity data.

4. The biological measurement apparatus according to any of claims 1-3, wherein the determination unit compares forces output by a plurality of the force sensor units with a determination value, and determines whether or not the forces are in a predetermined range.

5. The biological measurement apparatus according to any of claims 1-4, wherein the force sensor unit has a protrusion portion, and the protrusion portion protrudes from the infrared sensor unit toward the biological object.

6. The biological measurement apparatus according to any of claims 1-5, wherein the protrusion portion has an elastic body.

7. A biological measurement method comprising:
pressing an infrared sensor unit against a biological object;
detecting force with which the infrared sensor unit and the biological object are pressed together;
driving the infrared sensor unit when the force is in a predetermined range; and
irradiating, with the infrared sensor unit, the biological object with infrared light, receiving infrared light that was reflected by the biological object, and outputting light intensity data.

8. The biological measurement method according to claim 7, further comprising correcting the light intensity data using data indicating the force, the light intensity data, and a table indicating a relationship between the force and a correction amount for the light intensity data.
